# EUROPEAN PATENT SPECIFICATION

(11) **EP 0 684 472 B1**
(45) Date of publication and mention of the grant of the patent: **08.01.2003**
(21) Application number: 94931182.3
(22) Date of filing: 26.10.1994
(51) Int. Cl.: G01N 33/543

(54) **IMMUNOASSAY METHOD AND APPARATUS THEREFOR**
IMMUNOASSAY-VERFAHREN UND VORRICHTUNG DAFÜR
PROCEDE ET APPAREIL D'IMMUNOTITRAGE

(30) Priority: 26.10.1993 JP 26744693
(43) Date of publication of application: 29.11.1995
(73) Proprietor: NISSUI PHARMACEUTICAL CO., LTD., Toshima-ku, Tokyo 170-0002 (JP)
(72) Inventor: KUWADA, Atsushi, Daikin Industries, Ltd., Kusatsu-shi, Shiga 525 (JP); TOIDA, Tatsumi, Daikin Industries, Ltd., Kusatsu-shi, Shiga 525 (JP)
(74) Representative: Prüfer, Lutz H., Dipl.-Phys.
(86) International application number: JP9401822
(87) International publication number: WO95012126

(56) References cited:
- EP-A- 0 263 401
- JP-A- 62 148 858
- JP-A- 63 111 467

## Description

### Technical Field

The present invention relates to an immunoassay method and apparatus therefor. More particularly the present invention relates to an immunoassay method and apparatus therefor which measure a degree of immune reaction based upon a signal which corresponds to the quantity of labeled substances which are restrained by an immune reaction on the surface of a solid phase carrier, which solid phase carrier is obtained by making ligands to become a solid phase on a surface of a carrier, and which immunity reaction is carried out by pouring a liquid which includes a substance subjected to measurement and possibly other substances, and pouring a liquid containing ligands which are labeled with labeling substance.

### Background Art

From the past, an immunoassay method is proposed which includes carring out an immune reaction between a solid phase carrier and ligands within a liquid subjected to measurement, pouring a liquid which includes ligands labeled with labeling substance, and measuring a quantity of substance subjected to measurement included within the liquid for measurement based upon a signal which corresponds to the quantity of labeling substances which are restrained on a surface of the solid phase carrier by the immune reaction.

The immunoassay method is mainly classified into three methods depending upon the species of substance which are employed as the labeling substance.

A first method is an immunoassay method so called as EIA (Enzyme ImmunoAssay) which method employs an enzyme as the labeling substance. The method is exemplified as EIA, which method forms employs a plurality of liquid housing sections, in which ligands are previously fixed on a plate which is formed mainly of polystyrene. In the method, a liquid subjected to measurement, which liquid includes substance subjected to measurement, is poured into the liquid housing sections so that the ligands and the substance subjected to measurement are reacted with one another. Thereafter, liquid which includes ligands labeled with enzyme is poured into the liquid housing sections. Then, a substrate is reacted with the enzyme restrained in the liquid housing sections, which substrate changes its absorbance when it reacts with the enzyme. A light is irradiated to the liquid housing sections and the quantity of the substance subjected to measurement is measured depending upon the intensity of the transmitted light.

A second method is an immunoassay method so called as RIA (Radio ImmunoAssay), which method employs radioactive substance as the labeling substance. The method is exemplified as RIA, which method employs previously fixing ligands onto beads which is formed mainly of polystyrene. In the method, a liquid subjected to measurement, which liquid includes substance subjected to measurement, is poured so as to react the ligands with the substance subjected to measurement. Thereafter, ligands which are labeled with radioactive substance such as I125 are poured. The substance subjected to measurement react with the ligands labeled with the radioactive substance so that the radioactive substance are restrained to the beads. The quantity of the substance subjected to measurement is measured by measuring the radiant ray which is radiated from the radioactive substance restrained to the beads.

A third method is an immunoassay method so called as FIA (Fluorescence ImmunoAssay) which method empolys a fluorescent substance as the labeling substance. The method is exemplified as FIA, which method employs an immune reaction between ligands which are previously fixed to beads which are formed mainly of polystyrene, or ligands previously fixed on a surface of an optical waveguide, and ligands within a liquid subjected to measurement. The method further employs an immune reaction between ligands labeled with a fluorescent dye and the reacted ligands. Thereafter, in the method an exciting light is introduced so that the exciting light propagates within the optical waveguide in a totally reflective manner so as to generate an evanescent wave component. The fluorescent dye restrained on a surface of the optical waveguide is excited using the evanescent wave component. In the method, the degree of immune reaction is measured based upon the intensity of the excited fluorescence.

In these immunoassay methods, a method is generally employed, in which a liquid including substance subjected to measurement is poured on the surface of a solid phase carrier which is obtained by bringing ligands into a solid phase condition on the surface of a carrier. Thereafter, ligands labeled with labeling substance are poured. In the method, a degree of immune reaction is measured based upon a signal which corresponds to the quantity of the labeling substances which are restrained on the surface of the solid phase carrier by the immune reaction. Hereinafter, the method is referred to as a 2 steps method.

When the 2 steps method is employed, a disadvantage arises in that the same measurement results to one another may be obtained in cases that ligands within a liquid subjected to measurement have a high concentration and that ligands within a liquid subjected to measurement have low concentration. By taking a condition into consideration, which condition is that a degree of immune reaction is measured as a pretreatment of a medical care and the like in many cases, erroneous measurement results may have bad influences on the human body, so that this disadvantage cannot be ignored at all.

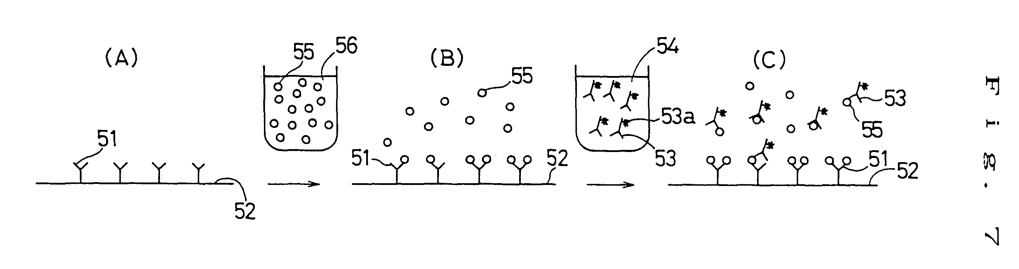

Occurence of the disadvantage is described in detail with reference to Fig. 7 on the basis of an example in which FIA is employed and antigens and antibodies are employed as ligands.

In this case of the 2 steps method, a liquid subjected to measurement 56, which includes antigens 55, is poured on the surface of an optical waveguide 52 on which antibodies 51 are previously fixed, as is illustrated in Fig. 7(A). Thereafter, a reagent liquid 54 is poured on the surface of the optical waveguide 52, which reagent liquid 54 includes labeled antibodies 53 which are obtained by labeling antibodies with fluorescent dye 53a. In this case, the concentration of the antigens 55 within the liquid subjected to measurement 56 is unknown. Therefore, there may occur a condition that the antigens 55 are excessive with respect to the labeled antibodies 53, a condition that the antibodies 53 are excessive with respect to the antigens 55, or a condition that the antigens 55 and labeled antibodies 53 are almost equilibrium to one another. When the antigens 55 are excessive, antigens 55 which do not contribute antigen-antibody reaction exist fairly much on the surface of the optical waveguide 52 prior to pouring of the reagent liquid 54 {refer to Fig. 7(B)}. When the reagent liquid 54 is poured thereafter, the labeled antibodies 53 perform antigen-antibody reaction with not only the antigens 55 which have already performed antigen-antibody reaction with the antibodies 51 fixed on the surface of the optical waveguide 52 but also the antigens 55 which are floating in the vicinity of the surface of the optical waveguide 52, as is illustrated in Fig. 7(C). Such phenomenon is called as a High Dose Hook phenomenon. When the quantity of the labeled antibodies 53 is increased, which labeled antibodies react with the antigens 55 which do not perform antigen-antibody reaction with the fixed antibodies 51, such labeled antibodies 53 cannot perform reaction with the antigens 55 which have already performed antigen-antibody reaction with the antibodies 51. Therefore, the quantity of the labeled antibodies 53 becomes smaller, which labeled antibodies are restrained in the vicinity of the surface of the optical waveguide 52, so that it becomes impossible that the concentration of the antigens 55 within the liquid subjected to measurement is detected uniquely.

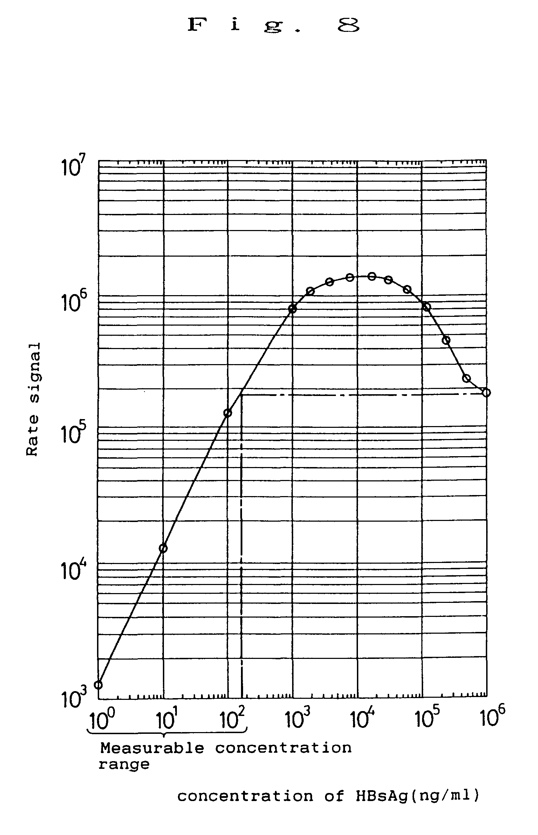

Fig. 8 is a graph illustrating an analytical curve of an example when the 2 steps method is employed and a rate method is employed, which rate method measures the fluorescence signal value for a determined time period and calculates a rate signal based upon a varying degree of the signal value. The horizontal axis represents a concentration of antigens (HBsAg) within a liquid subjected to measurement, while the vertical axis represents a rate signal based upon the rate method.

As is apparent from Fig. 8, when the concentration of the antigens exceeds about 1.0 X 10⁴ ng/ml, the rate signal is lowered due to the High Dose Hook phenomenon so that two concentrations of the antigens correspond to one rate signal value.
Therefore, it is necessary that the concentration measurement is carried out within an extent in which one concentration of the antigen corresponds to one rate signal value, for maintaining the accuracy in the concentration measurement. As a result, the disadvantage arises in that a measurable extent in concentration (measurement range) becomes narrower. Specifically, the concentration of HBsAg, illustrated in Fig. 8, can be measured up to about 2.0 X 10² ng/ml at the highest in the high concentration region. For example, the concentration of the HBsAg, which are antigens of B-type hepatitis, is about 0 ng/ml for a normal human, and the abnormal concentration of the HBsAg exceeds 2.0 X 10² ng/ml greatly. It is a great problem for immunoassay that the extent of measurable concentration is narrow.

The problem is solved by employing a method which performs antigen-antibody reaction between the antigens or antibodies which are fixed on a surface and the antibodies. In the method, the liquid is discharged for measurement. The method performs antigen-antibody reaction between the labeled antigens or the labeled antibodies and the antibodies or antibodies which have already performed antigen-antibody reaction. When the immunoassay method is employed, a processing for discharging the liquid subjected to measurement becomes necessary. Consequently, the time period required for the immunoassay becomes longer and the processings become complicated when the processings for immunoassay are automated. Especially, the disadvantage of legthening the measurement time period becomes remarkable when a plurality of measurement items are measured in an apparatus for immunoassay. Further, when the liquid subjected to measurement is imperfectly discharged, a disadvantage arises in that the liquid including labeled antigens or labeled antibodies is diluted so that the accuracy in immunoassay is lowered.

Further, problems due to the High Dose Hook phenomenon are generated in a 1 step sandwich method similarly to the 2 steps method. In the 1 step sandwich method, a reagent liquid and the liquid subjected to measurement including antigens are previously mixed. The mixed liquid is poured on the surface of the optical waveguide on which antibodies are previously fixed. Furthermore, problems due to the High Dose Hook phenomenon are generated in a method which performs third reaction and fourth reaction sequentially, wherein both reactions utilizing antigen-antibody reaction so that the labeling substance are restrained in the vicinity of a surface of an optical waveguide and the degree of immune reactions is measured, similarly to the 2 steps method.

Further, disadvantages similar to the above disadvantages arise when one of various immunoassay methods such as EIA, RIA and the like is employed.

From JP 63111467 an immunoassay is known, wherein a labeling antibody is added to a microtiter plate and lyophilized and a sample is added thereto.

EP 0 263 401 discloses an immunometric determination method which allows for detecting the Hook effect.

### Disclosure Of The Invention

The present invention was made to solve the above problems.

It is an object of the present invention to offer an immunoassay method and apparatus therefor which enlarge the measurable extent of the degree of immune reaction in various immunoassay, and shorten the measurement time period by omitting discharging processing of a liquid subjected to measurement.

To perform the above object, the present invention provides according to claim 1 an immunoassay method which comprises the steps of (a) fixing ligands on the surface of a carrier, whereby a solid phase carrier comprising the ligands is formed, (b) pouring a liquid containing ligands which are labeled with a labeling substance onto the surface of said solid phase carrier, (c) pouring a liquid containing a substance to be assayed in the immunoassay gradually onto said solid phase carrier and the liquid, (d) measuring the degree of the immune reaction on the basis of a signal which corresponds to the quantity of the ligands being labeled with the labeling substance which are restrained in the vicinity of the surface of the solid phase carrier by the immune reaction.

The carrier is exemplified by polystyrene beads, a test tube, a micro titration plate, a nitrocellulose sheet, an optical waveguide and the like. The ligand is exemplified by an antigen, an antibody, a hapten, a hormone, and an organic substance which cause specific bonding reaction.

In a preferred embodiment of the immunoassay method, the liquid including the substance subjected to measurement is poured by a quantity per unit time, which quantity is smaller than the pouring quantity of the liquid including ligands which are obtained by labeling antibodies with a labeling substance.

To perform the above object, an immunoassay apparatus according to claim 7 comprises,
(a) pouring means for pouring a liquid containing ligands which are labeled with a labeling substance onto the surface of a solid phase carrier, on which ligands are fixed,
(b) pouring controlling means for gradually pouring a liquid containing a substance to be assayed in the immunoassay onto the above solid phase carrier and the liquid,
(c) measuring means for measuring the degree of the immune reaction on the basis of a signal which corresponds to the quantity of the ligands being labeled with a labeling substance which are restrained in the vicinity of the surface of the solid phase carrier by the immune reaction.

In a preferred embodiment of the immunoassay apparatus, controlling means are further included for pouring the liquid including the substance subjected to measurement by a quantity per unit time which quantity is smaller than the pouring quantity of the liquid including ligands which are labeled with a labeling substance.

As to the immunoassay method according to claim 1, the liquid including the ligands labeled with labeling substance is poured on the surface of the solid phase carrier, the liquid including the substance subjected to measurement is poured on the surface of the solid phase carrier. Therefore, the ligands corresponding to the substance subjected to measurement sequentially perform reaction with the ligands labeled with the labeling substance and approach to the surface of the solid phase carrier. The ligands corresponding to the substance subjected to measurement, which ligands perform reaction with the ligands fixed on the surface of the solid phase carrier, are probably the ligands which have already performed a reaction with the ligands labeled with labeling substance. Consequently, the High Dose Hook phenomenon is suppressed, in which phenomenon the ligands labeled with labeling substance become difficult to be restrained by the ligands which are fixed on the surface of the solid phase carrier. As a result, the measurable extent is enlarged, in which extent one signal value corresponds to one degree of immune reaction. Further, the method is applicable even when the antigen-antibody reaction is performed within a small housing vessel, in which the discharging of the liquid subjected to measurement would be difficult, because discharging processing of the liquid subjected to measurement is not needed at all. The method shortens the time period required for the immunoassay, because the time period for the discharging processing can be omitted, which processing is needed in the conventional methods.

In the preferred embodiment of the immunoassay method according to the invention, the liquid including the substance subjected to measurement is poured by a quantity per unit time, which quantity is smaller than that of the pouring quantity of the liquid including ligands which are labeled with a labeling substance. The method decreases the ratio of the ligands corresponding to the substance subjected to measurement with respect to the ligands which are labeled with the labeling substance. This preferred embodiment further improves the rate of the ligands corresponding to the substance subjected to measurement, which ligands perform a reaction with the ligands labeled with a labeling substance and approach to the surface of the solid phase carrier, with respect to the entire ligands corresponding to the substance subjected to measurement. Therefore, the High Dose Hook phenomenon are further suppressed and the measurable extent in which one signal value value corresponds to one degree of immunity reaction is further enlarged.

As to the immunoassay apparatus according to claim 6, the pouring controlling means drives the pouring means so that the liquid including the ligands which are labeled with labeling substance are poured on the surface of the solid phase carrier, and the liquid including the substance subjected to measurement is poured on the surface of the solid phase carrier thereafter. The measuring means measures the degree of immune reaction based upon the signal corresponding to the quantity of the labeling substance which is restrained in the vicinity of the surface of the solid phase carrier by the antigen-antibody reaction. In this case, the apparatus improves the rate of the ligands corresponding to the substance subjected to measurement, which ligands perform a reaction with the ligands labeled with labeling substance and then approach to the surface of the solid phase carrier, with respect to the entire ligands corresponding to the substance subjected for measurement. Therefore, the apparatus suppresses the High Dose Hook phenomenon and further enlarges the measurable extent in which one signal value corresponds to one degree of immune reaction. Further, the apparatus is applicable even when the antigen-antibody reaction is performed within a small housing vessel, in which the discharging of the liquid subjected to measurement would be difficult, so that the immunoassay apparatus can be made small sized, because discharging processing of the liquid subjected to measurement is not needed at all. The apparatus is simplified in its arrangement when the apparatus is automated, because the pouring apparatus having a discharging processing function may not be considered at all. The apparatus shortens the time period for the immunoassay because the time period for the discharging processing may be omitted, which processing is needed in the conventional methods.

As to the preferred embodiment of the immunoassay apparatus according to the invention, the controlling means controls the pouring means so that the pouring means pours the liquid including the substance subjected to measurement by a quantity per unit time, which quantity is smaller than that of the pouring quantity of the liquid including ligands which are labeled with a labeling substance. The apparatus decreases the ratio of the ligands corresponding to the substance subjected to measurement with respect to the ligands which are labeled with the labeling substance. The apparatus further improves the rate of the ligands corresponding to the substance objected for measurement, which ligands perform a reaction with the ligands labeled with the labeling substance and approach to the surface of the solid phase carrier, with respect to the entire ligands corresponding to the substance subjected to measurement. Therefore, the apparatus further suppresses the High Dose Hook phenomenon and further enlarges the measurable extent in which one signal value corresponds to one degree of immunity reaction.

### Brief Description Of The Drawings

Figures 1(A) through 1(E) and 1(1) through 1(4) are diagrams schematically explaining an embodiment of the immunoassay method according to the present invention;
Figure 2 is a flowchart explaining the embodiment of the immunoassay·method according to the present invention;
Figure 3 is a chart illustrating an analytical curve of an example which is obtained by the embodiment of the immunoassay method;
Figure 4 is a flowchart explaining another embodiment of an immunoassay method according to the prsent invention;
Figure 5 is a block diagram illustrating a schematic arrangement of an embodiment of an immunoassay apparatus according to the present invention;
Figure 6 is a cross sectional view of a nozzle;
Figures 7(A) through 7(C) are diagrams schematically explaining the method for an optical immunoassay which employs a conventional 2 steps method; and
Figure 8 is a chart illustrating an analytical curve of an example which is obtained by the rate method employing a conventional 2 steps method.

### Best Modes For Performing The Invention

Hereinafter, referring to the attached drawings, we explain the present invention in detail.

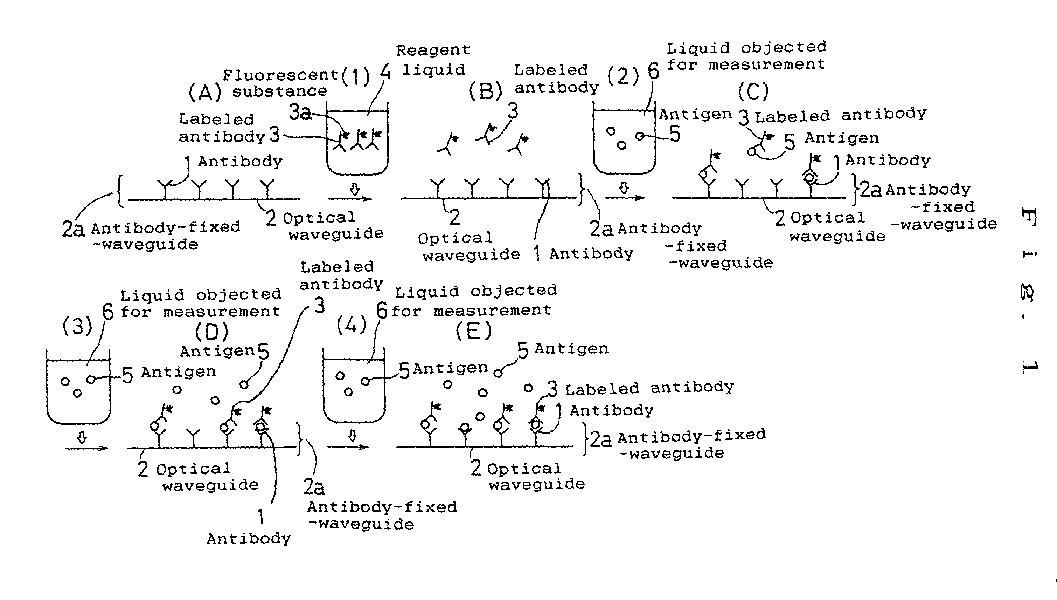

Figures 1(A) through 1(E) and 1(1) through 1(4) are diagrams schematically explaining an embodiment of an immunoassay method according to the present invention.

A reagent liquid 4 {refer to Fig. 1(1)} including labeled antibodies 3 which are obtained by labeling antibodies with a fluorescent substance 3a, is poured on a surface of an optical waveguide 2 on which antibodies 1 are previously fixed, as is illustrated in Fig. 1(A) (the optical waveguide 2, on which antibodies 1 are fixed as ligands, is referred to as antibody-fixed-waveguide 2a), so that a condition is obtained in which condition both antibodies 1 of the antibody-fixed-waveguide 2a and the labeled antibodies 3 exist, as is illustrated in Fig. 1(B). Then, a liquid 6 subjected to measurement including antigens 5, as is illustrated in Fig. 1(2), is poured by every small quantity. In this case, the quantity of the antigens 5 included in the liquid 6 objected for measurement is not known. But, the generation of the High Dose Hook phenomenon is suppressed so that most of antigens 5 perform an antigen-antibody reaction with floating labeled antibodies 3, and then perform an antigen-antibody reaction with the antibodies 1 of the antibody-fixed-waveguide 2a {refer to Fig. 1(C)}. Further, Figs. 1(3) and 1(D) and Figs. 1(4) and 1(E) represent conditions in which the pouring processing of the liquid 6 objected for measurement is advanced, respectively. The pouring processing of the liquid 6 objected for measurement is continued until the pouring of a predetermined quantity of the liquid 6 subjected to measurement has been finished.

After the pouring of the reagent liquid 4 including the labeled antibodies 3 on the surface of the antibody-fixed-waveguide 2a, the liquid 6 subjected to measurement is poured so that the antigens 5 sequentially perform a reaction with the labeled antibodies 3, and then approach to the surface of the antibody-fixed-waveguide 2a. Therefore, the rate of the antigens 5, which perform reaction with the antibodies 1 fixed on the surface of the antibody-fixed-waveguide 2a and which have already performed a reaction with the labeled antibodies 3, becomes higher. Consequently, the High Dose Hook phenomenon is suppressed so that it is prevented that the same rate signals are obtained when the concentration of the antigens is high and when the concentration of the antigens is low. Further, in the method the liquid 6 subjected to measurement including the antigens 5 is poured by every small quantity so that a condition is generated in which the number of the antigens 5 is small with respect to the labeled antibodies 3 within the liquid in the initial stage of pouring of the liquid 6 objected for measurement. The High Dose Hook phenomenon is further suppressed. Further, when the antigens 5 are sandwiched between the previously fixed antibodies 1 and the labeled antibodies 3, an antigen-antibody reaction causing the High Dose Hook phenomenon never occurs even when much quantity of antigens 5 is poured thereafter. Therefore, a rate signal corresponding to an essential quantity of the antigens 5 within the liquid 6 subjected to measurement does not change so that the concentration of the antigens 5 is uniquely detected up to a high concentration range. Further, the time period for immunoassay is shortened because a discharging of the liquid at every antigen-antibody reaction is not needed at all, which liquid faces the antibody-fixed-waveguide 2a.

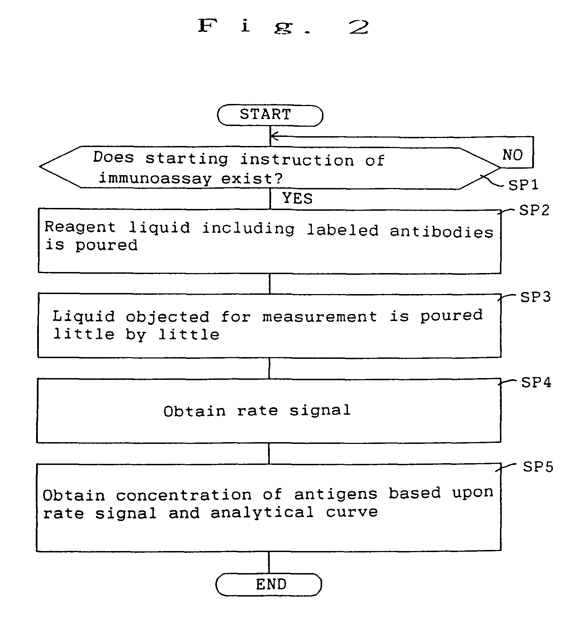

Fig. 2 is a flowchart explaining the embodiment of the immunoassay method according to the present invention. The method employs a reaction vessel which has an antibody-fixed-waveguide.

In step SP1, a starting instruction of an optical immunoassay is a waited. In step SP2, the reagent liquid including labeled antibodies, which are obtained by labeling antibodies with a fluorescent substance, is poured within the reaction vessel. In step SP3, the liquid subjected to measurement including the antigens is poured in a condition that the pouring quantity per second is decreased. In step SP4, the signal value is measured for a predetermined time period, and the rate signal is calculated based upon varying rate of the signal value. In step SP5, the concentration of the antigens within the liquid subjected to measurement is obtained based upon the calculated rate signal and the previously obtained analytical curve.

A method for calculating the concentration of the antigens using a so called end-point method may be employed instead of the method for otaining the concentration of the antigens using the rate signal. The end-point method calculates a degree of immune reaction based upon intensity of fluorescence, which intensity is detected at a timing when the intensity of the fluorescence becomes nearly stable.

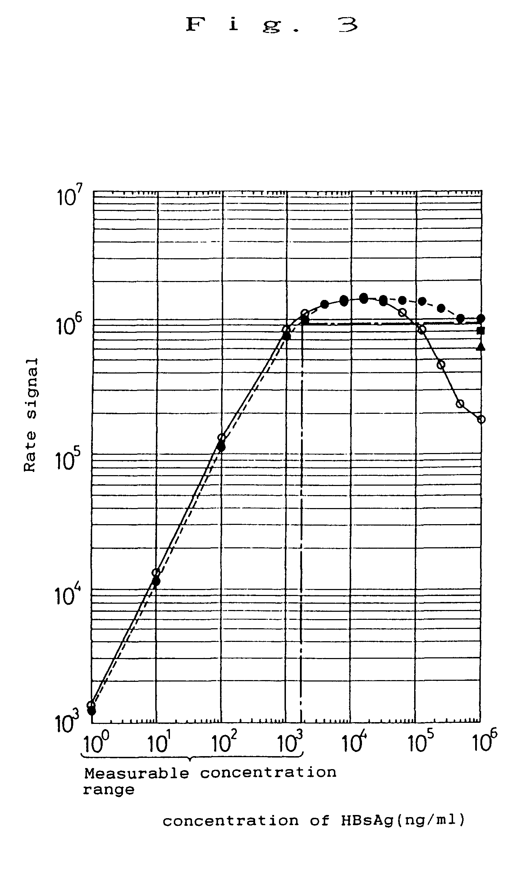

Fig. 3 is a chart illustrating an analytical curve of an example which is obtained by the embodiment of the immunoassay method. The chart represents an analytical curve with black circles, which analytical curve corresponds to a case in which the reagent liquid including the labeled antibodies which are obtained by labeling antibodies with fluorescent substance is poured into the reaction vessel, and thereafter the liquid subjected to measurement including antigens is poured by determining the pouring quantity to be 500 pps (pulse per second). The speed for pouring the reagent liquid is determined to be 3000 pps, therefore the reagent liquid is poured by 0.017 µl for 1 pulse of a pulse motor. Further, black rectangles represent data which correspond to a case in which the reagent liquid including the labeled antibodies which are obtained by labeling antibodies with fluorescent substance is poured into the reaction vessel, and thereafter the liquid subjected to measurement including antigens is poured by determining the pouring quantity to be 1000 pps. Black triangles represent data which correspond to a case in which the reagent liquid including the labeled antibodies which are obtained by labeling antibodies with fluorescent substance, is poured into the reaction vessel, and thereafter the liquid subjected to measurement including antigens is poured by determining the pouring quantity to be 3000 pps. Further, white circles represent an analytical curve of a conventional method which pours the liquid subjected to measurement and thereafter pours the reagent liquid by determining the pouring quantity to be 3000 pps.

From Fig. 3, it is understood that one concentration value corresponds to one rate signal even when the concentration becomes higher so that a measurable range is enlarged, when the reagent liquid including the labeled antibodies which are obtained by labeling antibodies with fluorescent substance is poured and when the liquid subjected to measurement is poured thereafter by every small quantity. Specifically, the conventional example illustrated by white circles can measure a high concentration range of up to about 2.0 X 10² ng/ml. To the contrary, the embodiment according to the invention can measure a high concentration range of up to about 2.0 X 10³ ng/ml when the liquid subjected to measurement including . antigens is-poured by determining the pouring quantity from the nozzle to be 500 pps (black circles). Further, from the data corresponding to the pouring quantity which is determined to be 3000 pps (black triangles), 1000 pps (black rectangles), 500 pps (black circles), it is understood that when the reagent liquid including labeled antibodies, which are obtained by labeling antigens with fluorescent substance, is poured and thereafter the liquid subjected to measurement including antigens is poured, the measurable range is enlarged by gradually pouring the liquid subjected to measurement by every small pouring quantity.

### Second Embodiment

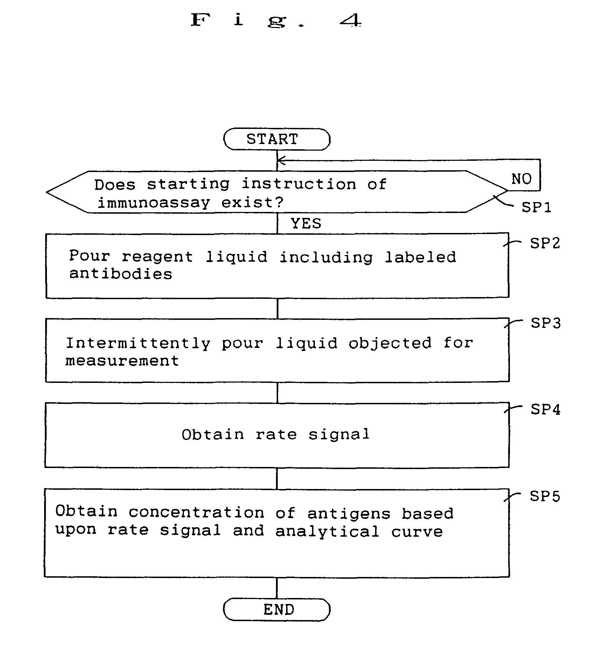

Figure 4 is a flowchart explaining another embodiment of an immunoassay method according to the present invention.

This embodiment differs from the first embodiment in that the liquid objected for measurement is intermittently poured so that the pouring quantity per unit time period is decreased. That is, the pouring of the liquid subjected to measurement in step SP3 in Fig. 2 is performed intermittently by a predetermined time interval so that the pouring quantity per unit time period is decreased. Further, the processings after the step SP3 are the same to the flowchart illustrated in Fig. 2. This embodiment obtains effects which are similar to that of the previous embodiment which varies the number of pulses of the pulse motor so that the pouring quantity from the nozzle is decreased.

### Third Embodiment

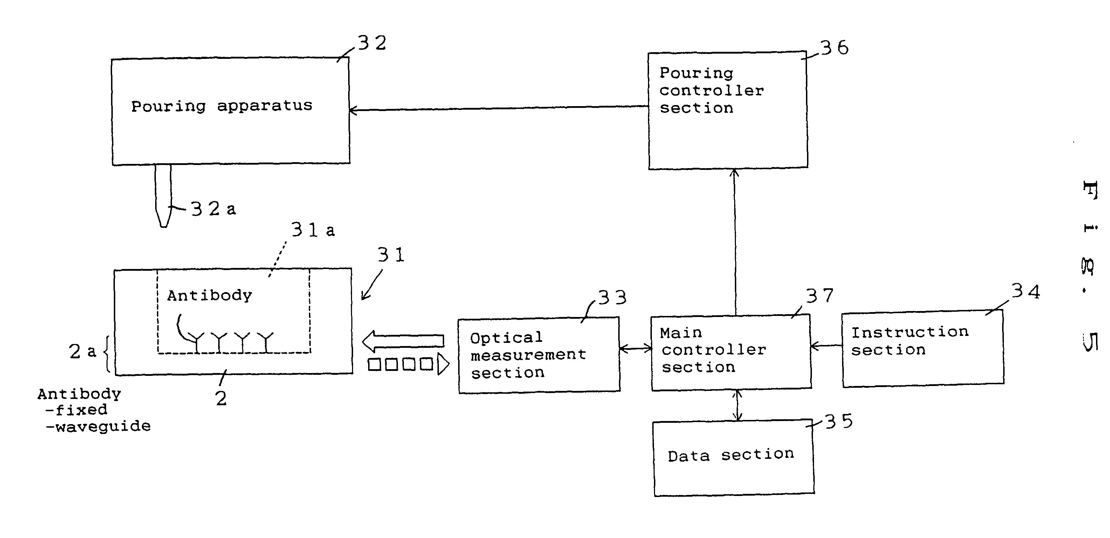

Figure 5 is a block diagram illustrating a schematic arrangement of an embodiment of an immunoassay apparatus according to the present invention.

The optical immunoassay apparatus includes a cell 31 for measurement, a pouring apparatus 32 for pouring a liquid into a predetermined vessel, an optical measurement section 33, an instruction section 34 to which user's instruction is input, a data section 35 for holding predetermined data corresponding to conditions such as inspection target, measurement item, the number of measurements and the like, a pouring controller section 36, and a main controller section 37. The cell 31 for measurement includes a reagent housing vessel for housing a reagent liquid including fluorescence labeled antibodies, a measurement liquid housing vessel for housing a liquid subjected to measurement, a diluting liquid housing vessel (foregoing vessels are not illustrated), a reaction vessel 31a for performing an antigen-antibody reaction, and an optical waveguide 2 (antibody-fixed-waveguide 2a) on which antibodies 1 are fixed, which waveguide 2 is formed on a predetermined face of the reaction vessel 31a. The optical measurement section 33 is provided at a predetermined location with respect to the cell 31 for measurement. The optical measurement section 33 introduces an exciting light into the optical waveguide 2. The optical measurement section 33 detects a signal light from the optical waveguide 2 so as to detect a degree of immune reaction. The pouring controller section 36 controls suction, dischargment, sucking quantity, and discharging quantity of the pouring nozzle 32a based upon the information of the instruction section 34 and the data section 35. The main controller section sends instructions to the optical measurement section 33 and the pouring controller section 36.

The operation of the optical immunoassay apparatus having the above arrangement is as follows.

When a user instructs an operation for predetermined imunoassay through the instruction section 34, the main controller section 37 draws out corresponding data from the data section 35 and drives the pouring controller section 36 based upon the data and the user's instruction. Specifically, the pouring controller section 36 drives the pouring nozzle 32a so as to suck the reagent liquid including fluorescence labeled antibodies from the reagent housing vessel and to pour the sucked reagent liquid into the reaction vessel 31 on which antibodies are fixed. Then, the pouring nozzle 32a sucks diluting liquid by a predetermined quantity from the diluting liquid housing vessel and sucks the liquid subjected to measurement from the measurement liquid housing vessel so as to dilute the liquid subjected to measurement. And, the pouring nozzle 32a pours the diluted liquid objected for measurement by every small quantity into the reaction vessel 31a so as to perform antigen-antibody reaction under a condition that the concentration of the antigens within the liquid objected for measurement is not too much with respect to the fluorescence labeled antibodies. Therefore, the concentration of the antigens within the liquid objected for measurement can be measured within a wider concentration range. Further, adjustment of sucking quantity of the liquid objected for measurement is performed by decreasing the number of pulses per second of the pulse motor by the pouring controller section 36 or by intermittently repeating the sucking and discharging by a predetermined quantity by the pouring controller section 36, so that the sucking quantity per a unit time period is decreased. The pulse motor is employed for adjusting the sucking quantity and discharging quantity of the pouring nozzle 32a.

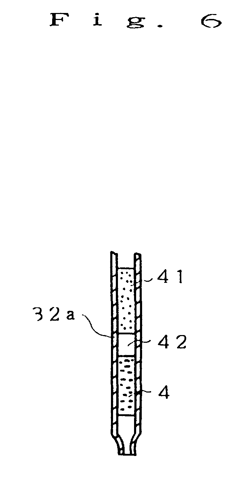

Further, when the reagent liquid 4 including the fluorescence labeled antibodies should be poured at a predetermined timing, and when the pouring nozzle 32a includes a liquid 41 which has already been sucked, as is illustrated in Fig. 6, the reagent liquid 4 is prevented from mixing with the liquid 41 by forming an air gap 42 between the already sucked liquid 41 and the reagent liquid 4. More than two liquids are sucked and discharged without mixing with one another by forming one or more air gaps 42 within the pouring nozzle 32a, so that a number of nozzles can be decreased and the measurement processing can be performed smoothly.

As is described above, the optical immunoassay apparatus according to this embodiment enlarges the measurable concentration extent (measurement range) of the liquid objected for measurement, and simplifies the arrangement of the apparatus because discharging processing of the liquid is not needed at all. Further, the apparatus has advantage in that the concentration of the liquid objected for measurement is measured with high accuracy and with a short time period.

The present invention is not limited to the above embodiments. Various design changes can be applied within an extent which does not vary the scope of the present invention.

For example, it is possible that the liquid objected for measurement including antibodies is gradually poured by every small quantity just before the finish of the pouring of the reagent liquid including labeled antibodies, instead of pouring the liquid subjected to measurement including antigens. after the pouring of the reagent liquid incuding labeled antibodies on the surface of the optical waveguide on which antibodies are fixed, which processing is described in the above embodiments. The former processing obtains effects which are similar to those of the processing in which the liquid subjected for measurement is poured after the pouring of the reagent liquid. Further, it is possible that the concentration of antibodies can be measured by fixing antigens on the optical waveguide.

The method according to the present invention can be applied to a case which restrains the labeling substance in the vicinity of the optical waveguide so as to measure the degree of immune reaction, which restraining is performed by third order reaction and fourth order reaction, wherein both reactions employ antigen-antibody reactions sequentially, instead of the case which restrains the antigens within the liquid subjected for measurement by the antibodies fixed on the optical waveguide and the fluorescence labeled antibodies in a sandwich condition, which processing is described in the above embodiments. Such a conventional example of immunoassay is described in the following. For example, antibodies are previously fixed on the surface of an optical waveguide. A liquid subjected to measurement is poured on the surface of the optical waveguide so as to perform an antigen-antibody reaction (primary reaction) between antigens (substance subjected to measurement) within a liquid subjected to measurement and the fixed antibodies. Thereafter, a liquid including antibodies which have a biotin group is poured on the surface of the optical waveguide so as to perform an antigen-antibody reaction (secondary reaction) between the antibodies having a biotin group and the antigens which are restrained on the surface of the optical waveguide. Then, a reagent liquid including reactants which have fluorescent dye and an avidin group is poured on the surface of the optical waveguide so as to perform a reaction between biotin and avidin (third order reaction), whereby the fluorescent dye are restrained on the surface of the optical waveguide. The quantity of the restrained fluorescent dye corresponds to the quantity of the antigens which are the substance subjected to measurement. Then, an exciting light is guided into the optical waveguide so as to measure the intensity of fluorescence and to measure the quantity of the antigens within the liquid subjected to measurement. The biotin has the characteristic that it reacts and bonds with plural avidin.

When the immunoassay is performed without discharging the liquid subjected to measurement after the primary reaction using such conventional immunoassay method, a High Dose Hook phenomenon may be generated. Therefore, when the method according to the present invention is employed, and the liquid subjected to measurement is gradually poured after the pouring of the liquid including antibodies which have biotin groups on the surface of the optical waveguide, the High Dose Hook phenomenon during the primary reaction and the secondary reaction is suppressed so that the measurable concentration range is enlarged. Further, the measurement time period is shortened in comparison to a case which discharges the liquid objected for measurement.

Further, the disadvantage arises in immunoassay method such as EIA, RIA and the like, which disadvantage is that an extent for measuring a degree of immunity reaction becomes narrower due to the High Dose Hook phenomenon, thereby it is apparent that the immunoassay method according to the present invention is applicable to the conventional immunoassay method.

### Industrial Application

The present invention is applicable to an immunoassay method and apparatus which pours at least a liquid including substance subjected to measurement and labeled with labeling substance on a surface of a solid phase carrier which is obtained by forming ligands into solid phase on a surface of a carrier. The present invention enlarges the measurable range and shortens the measurement time period.

## Claims

1. An immunoassay method comprising the steps of
(a) fixing ligands (1) on the surface of a carrier (2), whereby a solid phase carrier (2a) comprising the ligands (1) is formed,
(b) pouring a liquid (4) containing ligands (3) which are labeled with a labeling substance (3a) onto the surface of said solid phase carrier (2a),
(c) pouring a liquid (6) containing a substance (5) to be assayed in the immunoassay gradually onto said solid phase carrier (2a) and the liquid (4),
(d) measuring the degree of the immune reaction on the basis of a signal which corresponds to the quantity of the ligands (3) being labeled with the labeling substance (3a) which are restrained in the vicinity of the surface of the solid phase carrier (2a) by the immune reaction.

2. The immunoassay method according to claim 1, **characterized in that** said liquid (6) containing the substance (5) to be assayed is poured by a quantity per unit time which is smaller than the pouring quantity of the liquid (4) containing ligands (3) which are labeled with the labeling substance (3a).

3. The immunoassay method according to claim 1, **characterized in that** the ligands(1) and the ligands (3) which are labeled with a labeling substance (3a) are antigens, antibodies haptenes, hormones or organic substances which cause a specific bonding reaction.

4. The immunoassay method according to claim 1, **characterized in that** the substance (5) is an antigen and that the *ligands* (1) and the ligands (3) which are labeled with a labeling substance (3a) are antibodies which react with this antigen in an antigen-antibody reaction.

5. The immunoassay method according to one of the preceding claims, **characterized in that** the labeling substance (3a) is a fluorescent substance.

6. The immunoassay method according to one of the preceding claims, **characterized in that** the carrier (2) consists of polystyrene beads, a test tube, a micro titration plate, a nitrocellulose sheet or an optical waveguide.

7. An immunoassay apparatus comprising
(a) pouring means (32) for pouring a liquid (4) containing ligands (3) which are labeled with a labeling substance (3a) onto the surface of a solid phase carrier (2a), on which ligands (1) are fixed,
(b) pouring controlling means (35, 36, 37) for gradually pouring a liquid (6) containing a substance (5) to be assayed in the immunoassay onto said solid phase carrier (2a) and the liquid (4),
(c) measuring means for measuring the degree of the immune reaction on the basis of a signal which corresponds to the quantity of the ligands (3) being labeled with a labeling substance (3a) which are restrained in the vicinity of the surface of the solide phase carrier (2a) by the immune reaction.

8. The immunoassay apparatus according to claim 7, **characterized in that** said pouring controlling means (35, 36, 37) includes controlling means (35, 36, 37) for pouring the liquid (6) containing the substance (5) to be assayed by a quantity per unit time which is smaller than the pouring quantity of the liquid (4) containing ligands (3) which are labeled with the labeling substance (3a).

9. The immunoassay apparatus according to claim 7, **characterized in that** the carrier (2) consists of polystyrene beads, a test tube, a micro titration plate, a nitrocellulose sheet or an optical waveguide.

## Patentansprüche

1. Immuno-Assay-Verfahren mit den Schritten:
(a) Fixieren von Liganden (1) auf der Oberfläche eines Trägers (2), wodurch ein Festphasenträger (2a) mit den Liganden (1) gebildet wird,
(b) Strömen lassen einer Flüssigkeit (4), die Liganden (3) enthält, welche mit einer Markierungssubstanz (3a) markiert sind, auf die Oberfläche des Festphasenträgers (2a),
(c) Strömen lassen einer Flüssigkeit (6), die eine in dem Immuno-Assay zu prüfende Substanz (5) enthält, nach und nach auf den Festphasenträger (2a) und die Flüssigkeit (4),
(d) Messen des Ausmaßes der Immunreaktion auf der Basis eines Signals, welches der Menge der mit der Markierungssubstanz (3a) markierten Liganden (3) entspricht, die in der Nähe der Oberfläche des Festphasenträgers (2a) durch die Immunreaktion zurückgehalten werden.

2. Immuno-Assay-Verfahren gemäß Anspruch 1, **dadurch gekennzeichnet, daß** die Flüssigkeit (6), die die zu prüfende Substanz (5) enthält, in einer Menge pro Zeiteinheit strömen gelassen wird, die kleiner ist als die Strömungsmenge der Flüssigkeit (4), die Liganden (3) enthält, die mit der Markierungssubstanz (3a) markiert sind.

3. Immuno-Assay-Verfahren gemäß Anspruch 1, **dadurch gekennzeichnet, daß** die Liganden (1) und die mit einer Markierungssubstanz (3a) markierten Liganden (3) Antigene, Antikörper, Haptene, Hormone oder organische Substanzen sind, die eine spezifische Bindungsreaktion verursachen.

4. Immuno-Assay-Verfahren gemäß Anspruch 1, **dadurch gekennzeichnet, daß** die Substanz (5) ein Antigen ist und daß die Liganden (1) und die mit einer Markierungssubstanz (3a) markierten Liganden (3) Antikörper sind, die mit diesem Antigen in einer Antigen-Antikörper-Reaktion reagieren.

5. Immuno-Assay-Verfahren gemäß einem der vorangehenden Ansprüche, **dadurch gekennzeichnet, daß** die Markierungssubstanz (3a) eine fluoreszente Substanz ist.

6. Immuno-Assay-Verfahren gemäß einem der vorangehenden Ansprüche, **dadurch gekennzeichnet, daß** der Träger (2) aus Polystyrol-Kügelchen, einem Teströhrchen, einer Mikrotiterplatte, einem Nitrocelluloseblatt oder einem optischen Wellenleiter besteht.

7. Immuno-Assay-Vorrichtung mit:
(a) einer Einströmeinrichtung (32) zum Einströmen lassen einer Flüssigkeit (4), die Liganden (3), welche mit einer Markierungssubstanz (3a) markiert sind, enthält, auf die Oberfläche eines Festphasenträgers (2a), auf dem Liganden (1) fixiert sind,
(b) einer Einströmsteuereinrichtung (35, 36, 37) zum allmählichen Einströmen einer Flüssigkeit (6), die eine im ImmunoAssay zu prüfende Substanz (5) enthält, auf den Festphasenträger (2a) und die Flüssigkeit (4) ,
(c) einer Meßeinrichtung zum Messen des Ausmaßes der Immunreaktion auf der Basis eines Signals, welches der Menge der mit einer Markierungssubstanz (3a) markierten Liganden (3) entspricht, die in der Nähe der Oberfläche des Festphasenträgers (2a) durch die Immunreaktion zurückgehalten werden.

8. Immuno-Assay-Vorrichtung gemäß Anspruch 7, **dadurch gekennzeichnet, daß** die Einströmsteuereinrichtung (35, 36, 37) eine Steuereinrichtung (35, 36, 37) einschließt zum Einströmen der Flüssigkeit (6), die die zu prüfende Substanz (5) enthält, mit einer Menge pro Zeiteinheit, die kleiner ist als die Einströmmenge der Flüssigkeit (4), die Liganden (3) enthält, welche mit der Markierungssubstanz (3a) markiert sind.

9. Immuno-Assay-Vorrichtung gemäß Anspruch 7, **dadurch gekennzeichnet, daß** der Träger (2) aus Polystyrol-Kügelchen, einem Teströhrchen, einer Mikrotiterplatte, einem Nitrocelluloseblatt oder einem optischen Wellenleiter besteht.

## Revendications

1. Procédé d'immunodosage comprenant les étapes consistant à :
(a) fixer des ligands (1) sur la surface d'un support (2), en conséquence de quoi un support en phase solide (2a) comprenant les ligands (1) est formé,
(b) verser un liquide (4) contenant des ligands (3) qui sont marqués avec une substance de marquage (3a) sur la surface dudit support en phase solide (2a),
(c) verser un liquide (6) contenant une substance (5) devant être dosée dans l'immunodosage progressivement sur ledit support en phase solide (2a) et le liquide (4),
(d) mesurer le degré de la réaction immune sur la base d'un signal qui correspond à la quantité des ligands (3) qui sont marqués avec la substance de marquage (3a) qui sont confinés au voisinage de la surface du support en phase solide (2a) par la réaction immune.

2. Procédé d'immunodosage selon la revendication 1, **caractérisé en ce que** ledit liquide (6) contenant la substance (5) devant être dosée est versé en une quantité par unité de temps qui est inférieure à la quantité versée du liquide (4) contenant des ligands (3) qui sont marqués avec la substance de marquage (3a).

3. Procédé d'immunodosage selon la revendication 1, **caractérisé en ce que** les ligands (1) et les ligands (3) qui sont marqués avec une substance de marquage (3a) sont des antigènes, des anticorps, des haptènes, des hormones ou des substances organiques qui provoquent une réaction de fixation spécifique.

4. Procédé d'immunodosage selon la revendication 1, **caractérisé en ce que** la substance (5) est un antigène et que les ligands (1) et les ligands (3) qui sont marqués avec une substance de marquage (3a) sont des anticorps qui réagissent avec cet antigène dans une réaction antigène-anticorps.

5. Procédé d'immunodosage selon l'une des revendications précédentes, **caractérisé en ce que** la substance de marquage (3a) est une substance fluorescente.

6. Procédé d'immunodosage selon l'une des revendications précédentes, **caractérisé en ce que** le support (2) est constitué de billes de polystyrène, d'un tube à essai, d'une plaque de microtitrage, d'une feuille de nitrocellulose ou d'un guide d'onde optique.

7. Dispositif d'immunodosage comprenant :
(a) des moyens de versage (32) pour verser un liquide (4) contenant des ligands (3) qui sont marqués avec une substance de marquage (3a) sur la surface d'un substrat en phase solide (2a), sur lequel des ligands (1) sont fixés,
(b) des moyens de commande de versage (35, 36, 37) pour verser progressivement un liquide (6) contenant une substance (5) devant être dosée dans l'immunodosage sur ledit support en phase solide (2a) et le liquide (4),
(c) des moyens de mesure pour mesurer le degré de la réaction immune sur la base d'un signal qui correspond à la quantité des ligands (3) qui sont marqués avec une substance de marquage (3a) qui sont confinés au voisinage de la surface du support en phase solide (2a) par la réaction immune.

8. Dispositif d'immunodosage selon la revendication 7, **caractérisés en ce que** lesdits moyens de commande de versage (35, 36, 37) comprennent des moyens de commande (35, 36, 37) pour verser le liquide (6) contenant la substance (5) devant être dosée d'une quantité par unité de temps qui est inférieure à la quantité versée du liquide (4) contenant des ligands (3) qui sont marqués avec la substance de marquage (3a).

9. Dispositif d'immunodosage selon la revendication 7, **caractérisés en ce que** le support (2) est constitué de billes de polystyrène, d'un tube à essai, d'une plaque de microtitrage, d'une feuille de nitrocellulose ou d'un guide d'onde optique.
